# EUROPEAN PATENT SPECIFICATION

(11) **EP 2 874 521 B1**
(45) Date of publication and mention of the grant of the patent: **11.09.2019**
(21) Application number: 12881471.2
(22) Date of filing: 19.07.2012
(51) Int. Cl.: A41D 13/05, A42B 3/04, A61F 5/055

(54) **HEAD AND NECK SUPPORT DEVICE WITH LOW COLLAR**
KOPF- UND NACKENSTÜTZVORRICHTUNG MIT NIEDRIGEM KRAGEN
DISPOSITIF DE SUPPORT DE TÊTE ET DE COU AYANT UN COL BAS

(43) Date of publication of application: 27.05.2015
(73) Proprietor: Simpson Performance Products, Inc., New Braunfels, TX 78130 (US)
(72) Inventor: STILES, Mark, A., Atlanta, GA 30319 (US); DOWNING, James, R., Atlanta, GA 30341 (US)
(74) Representative: Klunker IP Patentanwälte PartG mbB
(86) International application number: PCT/US2012/047394
(87) International publication number: WO 2014/014465

(56) References cited:
- WO-A1-2009/133524
- KR-A- 20100 121 671
- US-A- 6 009 566
- US-A- 6 009 566
- US-A1- 2009 044 314
- US-A1- 2009 144 886
- US-A1- 2009 144 886
- US-A1- 2010 229 290
- US-A1- 2010 286 580

## Description

### FIELD OF THE INVENTION

The present invention relates generally to head and neck support devices, and more particularly to head and neck support devices with a low collar.

### BACKGROUND OF THE INVENTION

US 2009/0044314 A1 teaches a head and neck protector for use by a race car or other driver.

A head and neck support (HANS) device is a structure that is worn around the neck and over the shoulders of a race car driver while racing. The HANS device is intended to reduce the likelihood or severity of head and/or neck injuries in the event of a crash or collision. A typical HANS device is formed with a high collar, a left leg unit and a right leg unit connected to the high collar. The high collar is an upright member in the center of the HANS device that is positioned behind the driver's neck and the head when the device is worn. The left and right leg units engage the driver's shoulders and the upper part of the chest. The driver's helmet is tethered to the back of the high collar to reduce the forces being transmitted to the neck of the driver in the event of a head-on or front end collision. For such a high collar design, the centerline of the tethers is 70 mm or higher above the top of the legs, so that the tethers are approximately horizontal as they are connected to the helmet.

Additionally, for the high collar design of the HANS device, in order to accommodate different sized drivers, different seating angles and different headrests of vehicles, the HANS device is manufactured in a variety of shapes, sizes, and configurations. This requires race teams to purchase multiple different HANS devices and often leads to confusion for the driver who must figure out which HANS device he/she needs for proper safety and comfort. Furthermore, the high collar HANS device needs more material and has more weight.

Therefore, a heretofore unaddressed need exists in the art to address the aforementioned deficiencies and inadequacies.

### SUMMARY OF THE INVENTION

In one aspect, the invention relates to a head and neck support (HANS) device. In one embodiment, the HANS device includes a low collar having a left side, a right side, a central portion defined between the left side and the right side, and a pair of openings formed in the central portion and proximate to the left side and the right side, respectively.

The HANS device also includes a left leg having a top portion extending laterally and outwardly from the left side of the low collar, and a bottom portion extending curvedly and downwardly from the top portion, and a right leg having a top portion extending laterally and outwardly extending from the right side of the low collar, and a bottom portion extending curvedly and downwardly from the top portion.

The low collar, the left leg and the right leg integrally form a yoke structure therewith, such that the central portion of the low collar is operable to be positioned behind the neck of a driver, the top portion of the left leg is operable to engage the left shoulder of the driver, the bottom portion of the left leg is operable to engage the left chest area of the driver, the top portion of the right leg is operable to engage the right shoulder of the driver, and the bottom portion of the right leg is operable to engage the right chest area of the driver, respectively.

Additionally, the low collar is characterized with a distance between a horizontal line crossing the middle of the pair of openings of the low collar and a top end of the top portion of each of the left leg and the right leg. The distance is less than 70 mm.

The HANS device further includes a tether having a strap coupled to the low collar through the pair of openings of the low collar, and a pair of catches connected to two ends of the strap, where the catches of the tether are operable to attach to a helmet of the driver so as to engage the low collar with the helmet of the driver.

In one embodiment, the low collar is formed such that a portion of the strap of the tether is exposed at a rear surface of the low collar.

In one embodiment, the low collar has a recess formed on a rear surface of the low collar at the central portion of the low collar such that the pair of openings are located in the recess, and a peg member configured complementarily to the recess and operable to be received in the recess. As assembled, the strap of the tether passes through the pair of openings of the low collar and a portion of the strap is accommodated in the recess of the low collar, and the peg member is pegged into the recess, such that no portion of the strap of the tether is exposed at a rear surface of the low collar.

In one embodiment, the low collar has a curved notch formed on a bottom edge of the central portion. The notch is operable to accommodate the neck of the driver.

The low collar has a collar height that varies gradually from a central portion to the left and right sides. The collar height at the central portion is higher than that at the left and right sides.

In one embodiment, the HANS device may include a left cushion detachably attached to a bottom surface of the left leg, and a right cushion detachably attached to a bottom surface of the right leg. Each cushion is operable to provide a buffer action for the corresponding shoulder and chest area of the driver.

In one embodiment, the HANS device includes a left load-bearing thin layer formed on a top surface of the left leg, and a right load-bearing thin layer formed on a top surface of the right leg. Each load-bearing thin layer is formed of a friction material and is operable to resist the shoulder belts from slipping along the load bearing surfaces of the HANS device.

In one embodiment, a top surface of each of the left leg and the right leg is provided with a friction means. The friction means provides for better transference of force from the load bearing surfaces to the shoulder harness in a crash.

In one embodiment, each of the left leg and the right leg has an upward lip with a variable height extending upwardly from an exterior edge of the top portion of the corresponding leg, where the upward lip is operable to reduce the chance of the shoulder belt of the safety harness sliding off the HANS device.

In another embodiment, the HANS device includes at least one sleeve member attached to the rear surface of the low collar's central portion to define the pair of openings therewith. In this case, the strap of the tether is coupled to the low collar through the openings of the at least one sleeve member.

Accordingly, the low collar design of the HANS device, among other things, has many advantages over a high collar design. For example, the low collar design allows universal applications to all vehicles regardless of seating angles or human body types since there is no high collar situated between the back of the helmet and the headrest of the vehicles. The low collar design also allows unobstructed exit from a vehicle which can sometimes be a problem with a high collar design in some vehicles. In addition, the low collar design can reduce the material and therefore the weight of the HANS device, which is always desired by all racing drivers. Further, the low collar design with the concealed tether variation (as called the "subjugator") routing can keep the tether from rubbing on the headrest which can damage it in some situations.

This summary is provided to introduce a selection of concepts in a simplified form that are further described below in the detailed description. This summary is not intended to identify key features or essential features of the claimed subject matter, nor is it intended to be used to limit the scope of the claimed subject matter.

These and other aspects of the present invention will become apparent from the following description of the preferred embodiment taken in conjunction with the following drawings, although variations and modifications therein may be effected without departing from the scope of the invention as defined by the claims.

### BRIEF DESCRIPTION OF THE DRAWING FIGURES

The accompanying drawings illustrate one or more embodiments of the invention and, together with the written description, serve to explain the principles of the invention. Wherever possible, the same reference numbers are used throughout the drawings to refer to the same or like elements of an embodiment. The drawings do not limit the present invention to the specific embodiments disclosed and described herein. The drawings are not necessarily to scale, emphasis instead being placed upon clearly illustrating the principles of the invention.
FIG. 1 shows a perspective view of a HANS device according to a first embodiment of the present invention.
FIG. 2 shows another perspective view of the HANS device according to the first embodiment of the present invention.
FIG. 3 shows a front view of the HANS device according to the first embodiment of the present invention.
FIG. 4 shows a side view of the HANS device according to the first embodiment of the present invention.
FIG. 5 shows an exploding perspective view of a HANS device according to a second embodiment of the present invention.
FIG. 6 shows another perspective view of the HANS device according to the second embodiment of the present invention.
FIG. 7 shows an exploding perspective view of a HANS device according to a third embodiment of the present invention.
FIG. 8 shows another perspective view of the HANS device according to the third embodiment of the present invention.
FIG. 9 shows schematically a use of the HANS device according to the first embodiment of the present invention.

### DETAILED DESCRIPTION OF THE EMBODIMENTS

The present invention will now be described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that the same thing can be said in more than one way. Consequently, alternative language and synonyms may be used for any one or more of the terms discussed herein, and no special significance is to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

It will be understood that when an element is referred to as being "on" another element, it can be directly on the other element or intervening elements may be present. In contrast, when an element is referred to as being "directly on" another element, there are no intervening elements present. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present invention. Furthermore, relative terms, such as "lower" or "bottom" and "upper" or "top", may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower", can therefore, encompass both an orientation of "lower" and "upper", depending of the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

As used in the description herein and throughout the claims that follow, the meaning of "a", "an", and "the" includes plural reference unless the context clearly dictates otherwise. Also, as used in the description herein and throughout the claims that follow, the meaning of "in" includes "in" and "on" unless the context clearly dictates otherwise.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

As used herein, the terms "comprising," "including," "having," "containing," "involving," and the like are to be understood to be open-ended, i.e., to mean including but not limited to.

In this description, references to "one embodiment", "an embodiment", or "embodiments" mean that the feature or features being referred to are included in at least one embodiment of the technology. Separate references to "one embodiment", "an embodiment", or "embodiments" in this description do not necessarily refer to the same embodiment and are also not mutually exclusive unless so stated and/or except as will be readily apparent to those skilled in the art from the description. For example, a feature, structure, act, etc. described in one embodiment may also be included in other embodiments, but is not necessarily included. Thus, the present technology can include a variety of combinations and/or integrations of the embodiments described herein.

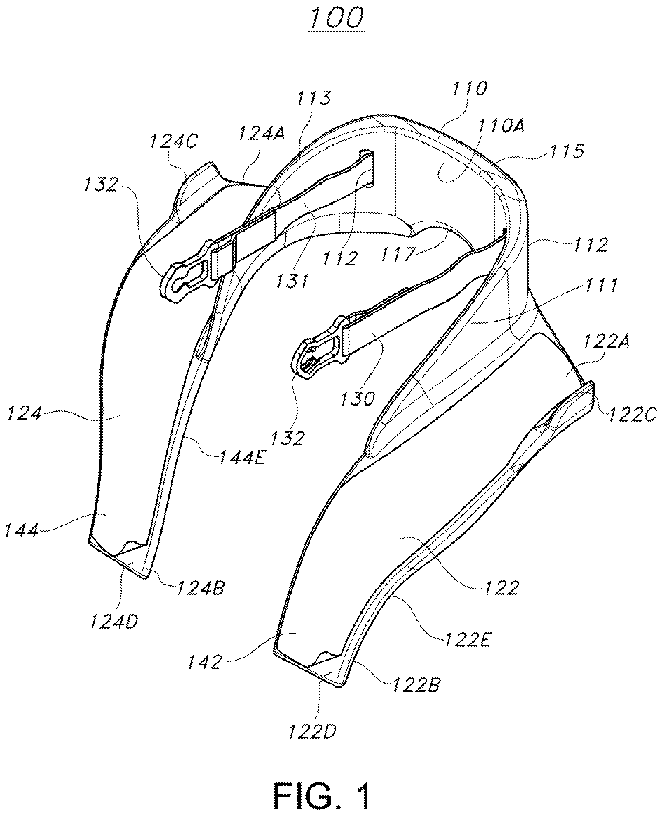

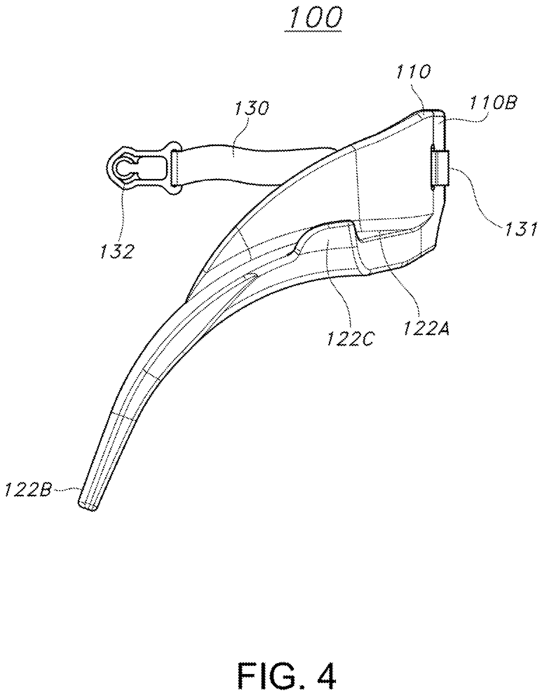

The description will be made as to the embodiments of the present invention in conjunction with the accompanying drawings in FIGS. 1-9. In accordance with the purposes of this invention, as embodied and broadly described herein, this invention, in one aspect, relates to a head and neck support (HANS) device having a low collar.

Referring to FIGS. 1-4, the HANS device 100 is shown according to one embodiment of the present invention. The HANS device 100 includes a low collar 110. The low collar 110 has a left side 111, a right side 113, a central portion 115 defined between the left side 111 and the right side 113, and a pair of openings 112 formed in the central portion 115 and proximate to the left side 111 and the right side 113, respectively.

The HANS device 100 also includes a left leg 122 and a right leg 124. The left leg 122 has a top portion 122A extending laterally and outwardly from the left side 111 of the low collar 110, and a bottom portion 122B extending curvedly and downwardly from the top portion 122A. The right leg 124 has a top portion 124A extending laterally and outwardly extending from the right side 113 of the low collar 110, and a bottom portion 124B extending curvedly and downwardly from the top portion 124A.

As shown in FIGS. 1-4, the low collar 110, the left leg 122 and the right leg 124 of the HANS device 100 integrally form a symmetrically U-shaped yoke structure therewith, such that the central portion 115 of the low collar 110 is operable to be positioned behind the neck of the driver, the top portion 122A of the left leg 122 is operable to engage the left shoulder of the driver, the bottom portion 122B of the left leg 122 is operable to engage the left chest area of the driver, the top portion 124A of the right leg 124 is operable to engage the right shoulder of the driver, and the bottom portion 124B of the right leg 124 is operable to engage the right chest area of the driver, respectively. Accordingly, the top portion 122A/124A of each leg 122/124 is also called a shoulder portion, and the bottom portion 122B/124B of each leg 122/124 is also called a chest portion. For each leg 122/124, the shoulder portion may be wider and thicker than the chest portion with a curvature on its bottom surface 122E/124E to match the shoulder of the driver.

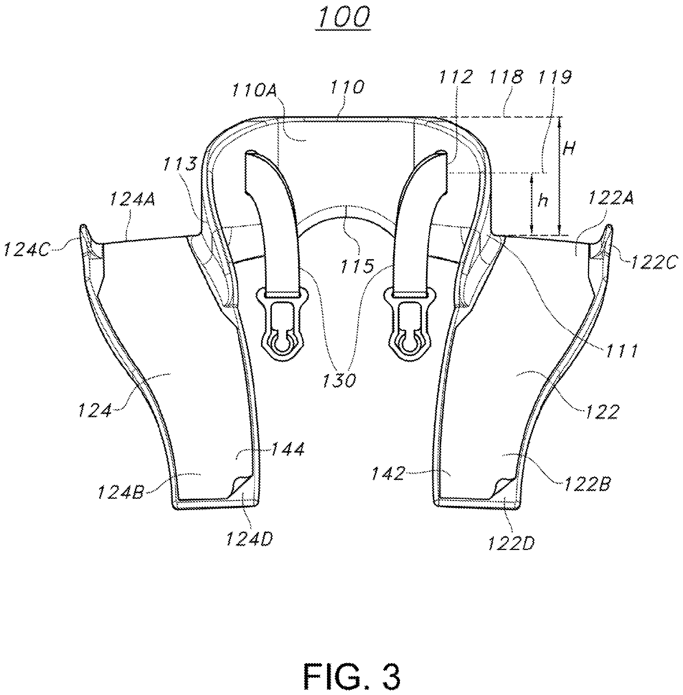

The low collar 110 is a generally upright member, and has a collar height, H, that varies gradually from a central portion 115 to the left and right sides 111 and 113. As shown in FIG. 3, the collar height H at the central portion 115 is higher than that at the left and right sides 111 and 113. The left and right sides 111 and 113 of the low collar 110 may taper forward from the central portion 115 such that the low collar 110 wraps around at least part of the neck of the driver. In other words, the low collar 110 is configured smoothly with a gradually reduced height along the left and right sides 111 and 113 so as to allow the driver to freely turn around his/her face to adjust his/her side vision if needed. And yet the left and right sides 111 and 113 is higher enough to operably reduce the chance of the shoulder belts of a shoulder harness slipping off the left and right legs 122 and 124 inwardly toward the neck of the driver.

The low collar 110 also has a curved or arched notch 117 formed on a bottom of the central portion 115, where the notch 117 is adapted for engaging the lower part of the neck and the upper part of the back of the driver. In addition, the bottom of the central portion 115 of the low collar 110 may have rounded edges for comfort.

As shown in FIGS 1-4, each of the left leg 122 and the right leg 124 has an upward lip 122C/124C extending upwardly from an exterior edge of the top portion 122A/124A of the corresponding leg 122/124. In this exemplary embodiment, the upward lip 122C/124C is formed with a variable height. The upward lip 122C/124C is operable to reduce the chance of the shoulder belt of the safety harness sliding off the left and right legs 122 and 124 of the HANS device 100.

Additionally, the HANS device 100 may include a left load-bearing thin layer/film 142 formed on the top surface 122D of the left leg 122, and a right load-bearing thin layer 144 formed on the top surface 124D of the right leg 124, as shown in FIGS. 1 and 3. Each load-bearing thin layer 142/144 is formed of a friction material and is operable to resist a shoulder belt of a safety harness from slipping on the left and right legs 122 and 124 of the HANS device 100. The friction material can be, for example, rubber.

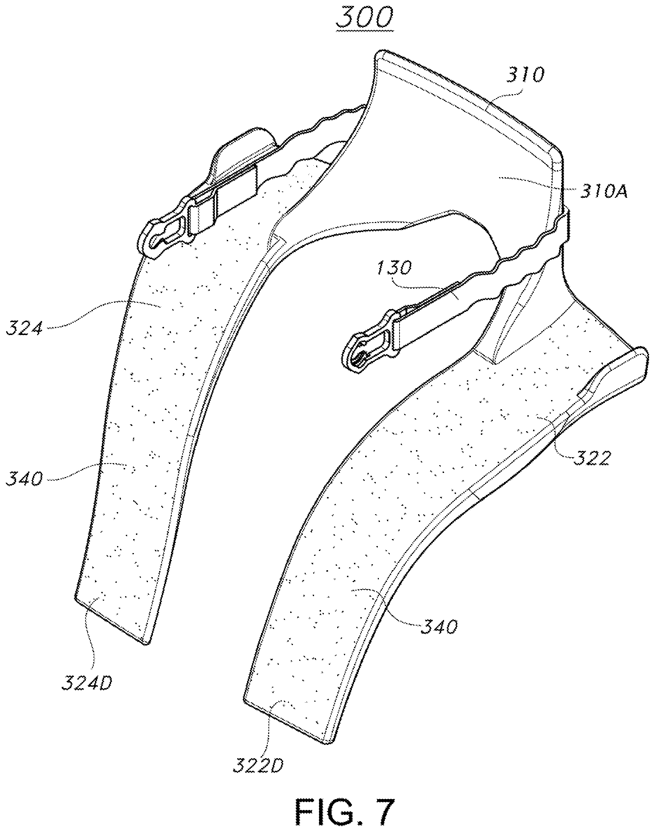

Alternatively, the top surface 122D/124D of each leg 122/124 is provided with a friction means. The friction means includes, for example, a pattern formed of a friction material such as rubber (as shown by 340 in FIG. 7). Similarly, the friction means is operable to resist the shoulder belts of the safety harness slipping on the left and right legs 122 and 124 of the HANS device 100.

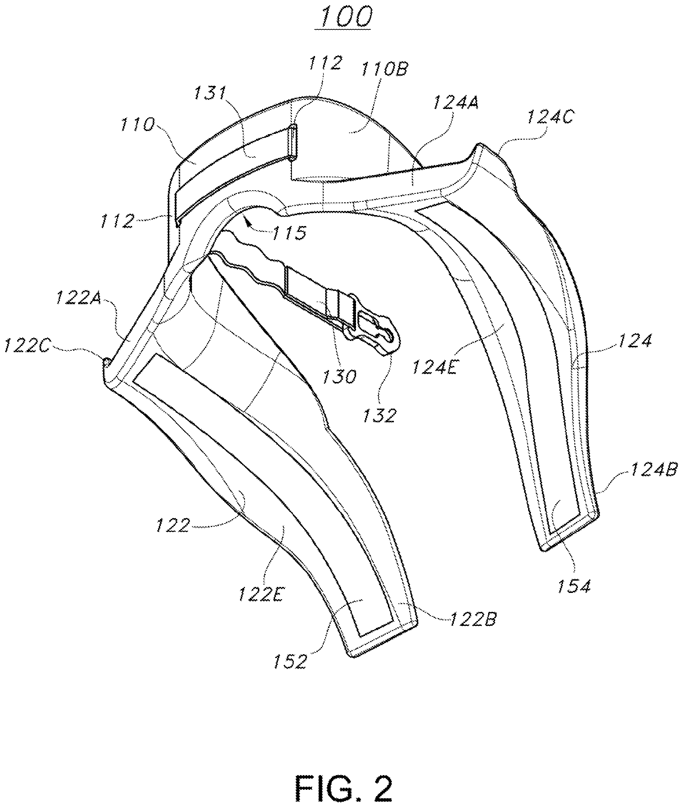

Furthermore, the HANS device 100 also includes a left cushion 152 attached to a bottom surface 122E of the left leg 122, and a right cushion 154 attached to a bottom surface 124E of the right leg 124, as shown in FIG. 2. Preferably, the cushion 152/154 is detachably attached to the bottom surface 122E/124E, as shown in FIG. 2. For example, the cushion 152/154 may be attached to the bottom surface 122E/124E by a hook and loop fastener, a snap fastener, or other attaching means. Each cushion 152/154 is operable to provide a buffer action for the corresponding shoulder and chest area of the driver.

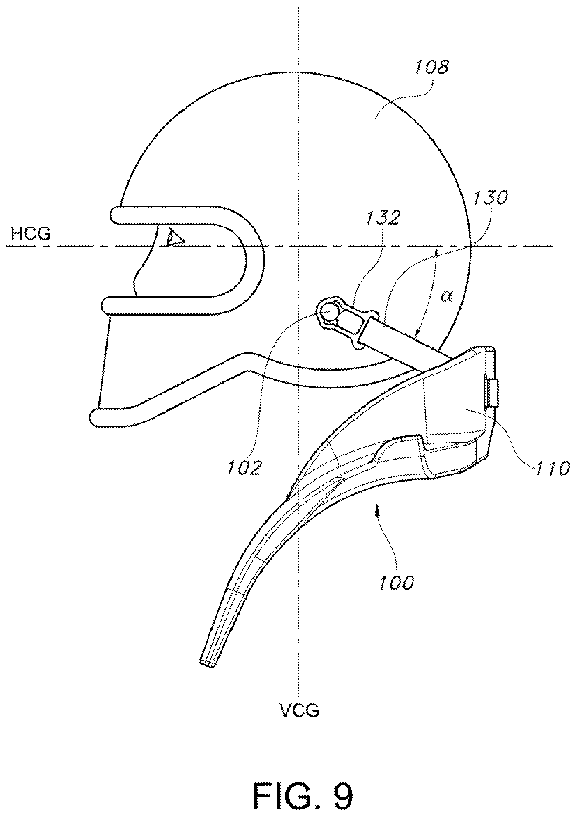

The HANS device 100 further includes a tether 130 having a strap 131 and a pair of catches 132 connected to two ends of the strap 131. The strap 131 is coupled to the low collar 110 through the pair of openings 112 of the low collar 110. In this exemplary embodiment shown in FIGS. 1-4, the strap 131 passes through the front surface 110A of the low collar 110, through one opening 112 of the low collar 110, extends along the rear surface 110B of the low collar 110, and passes through the other opening 112, such that a portion of the strap 131 of the tether 130 is exposed at the rear surface 110B of the low collar 110. The catches 132 are operable to attach to a helmet of the driver (for example, as shown in FIG. 9) so as to engage the low collar 110 with the helmet of the driver. In one embodiment, the tether 130 is connected in such a way as to allow side to side turning motion of the head of the driver. Preferably, the tether 130 has a fixed length. The length of the tether 130 is such as to allow some mobility of the driver's head. The length of the tether 130 preferably allows the driver to have the ability to rotate his/her head to increase his/her lateral sight area.

According to the invention, the HANS device 100 is a head and neck support device with the low collar 110 that is characterized with a distance, h, between a horizontal line 119 crossing the middle of the pair of openings 112 of the low collar 110 and a top end of the top portion 122A/124A of each of the left and right legs 122/124. The distance h is less than 70 mm.

The low collar design of the HANS device, among other things, has many advantages over a high collar design. For example, the low collar design allows universal applications to all vehicles regardless of seating angles or human body types since there is no high collar between the back of the helmet and the headrest of the vehicles. The low collar design also allows unobstructed exit from a vehicle which can sometimes be a problem with a high collar design in some vehicles. In addition, the low collar design can reduce the material used and therefore the weight of the HANS device, which is always desired by all racing drivers.

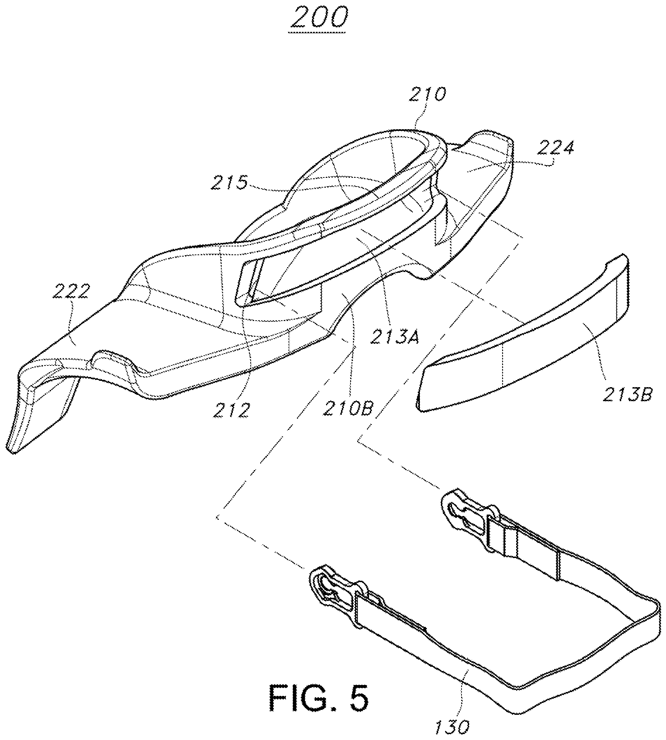

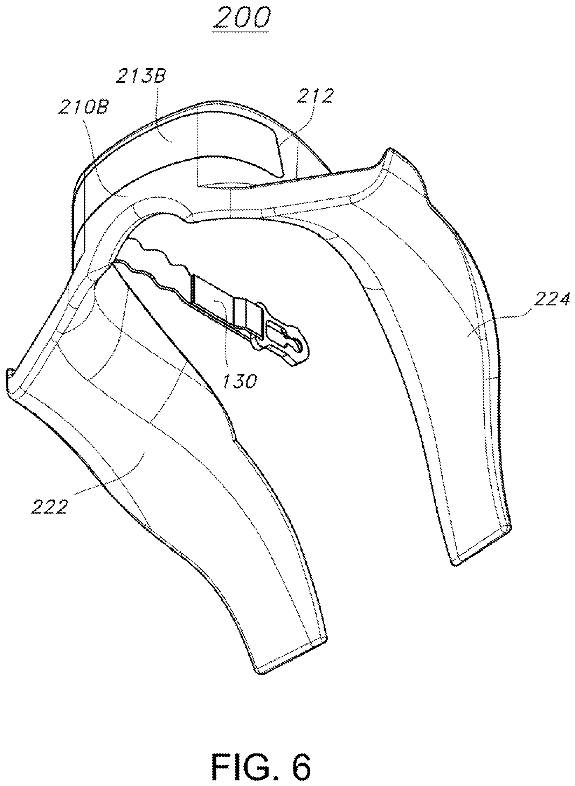

Referring to FIGS. 5 and 6, the HANS device 200 is shown according to another embodiment of the present invention. The HANS device 200 has a low collar 210 and a left leg 222 and a right leg 224 which integrally form a symmetrically U-shaped yoke structure therewith. The HANS device 200 is substantially the same as the HANS device 100 shown in FIGS. 1-4, except that the low collar 210 has a recess 213A formed on the rear surface 210B of the low collar 210 and a peg member 213B configured complementarily to the recess 213A and operable to be received in the recess 213A. The recess 213A is formed at the central portion 215 of the low collar 210 such that the pair of openings 212 is located in the recess 213A. As assembled, the strap 131 of the tether 130 passes through the pair of openings 212 of the low collar 210 and a portion of the strap 131 is accommodated in the recess of the low collar 210, and the peg member 213B is pegged into the recess 213A. As such, no portion of the strap 131 of the tether 130 is visually exposed in rear view of the rear surface 210B of the low collar 210. Accordingly, the low collar design with the concealed tether variation (as called the "subjugator") routing can keep the tether from rubbing on the headrest which can damage it in some situations.

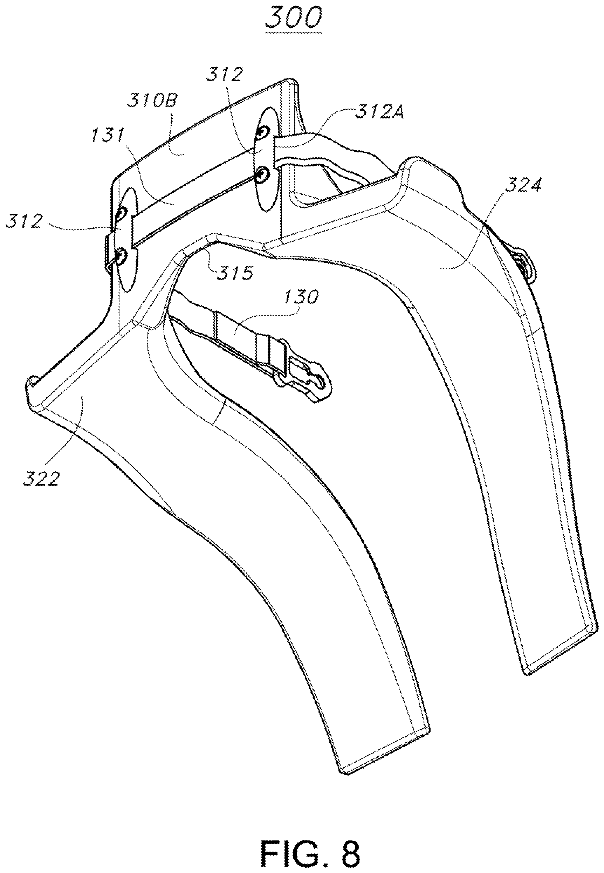

Referring now to FIGS. 7 and 8, the HANS device 300 is shown according to another embodiment of the present invention. The HANS device 300 has a low collar 310 and a left leg 322 and a right leg 324 integrally forming a symmetrically U-shaped yoke structure therewith. The HANS device 300 is substantially the same as the HANS device 100 shown in FIGS. 1-4, except that the low collar 310 has at least one sleeve member 312 attached to the rear surface 310B to define an opening 312A therewith. Preferably, the low collar 310 has two sleeve member 312 attached to the rear surface 310B of the low collar 310 at the central portion 315 of the low collar 310. As assembled, the strap 131 of the tether 130 passes through the opening 312A and extends along the rear surface 310B of the low collar 310. As such, a portion of the strap 131 of the tether 130 is exposed at the rear surface 310B of the low collar 310, but no portion of the strap 131 of the tether 130 passes through the front surface 310A of the low collar 310.

Additionally, the top of the central portion 315 of the low collar 310 is relatively flat, compared to the HANS devices 100 and 200 as disclosed above.

For the low collar design, the distance between a horizontal line crossing the middle of the openings of the sleeve members 312 and a top end of the top portion of each leg 322/324 is less than 70 mm.

According to the invention, the HANS device is generally rigid and may be manufactured from a lightweight, durable, stiff and relatively inflexible material, such as a material that can be formed into a hard mold or injected into a hard mold such as injection molded metal alloy material, a formable composite material, or a high impact resistant plastic material, such as formable carbon fiber or thermal set composite material. An exemplary HANS device may be manufactured from thixentropic injection molded magnesium, "THIXO", that includes a range of alloys such as AZ91, AM60, and the like. The THIXO alloys may include parameters such as a tensile strength of approximately 35,000 to approximately 40,000 psi (241 - 276 MPa), a stiffness (modulus) of approximately 7,500,000 psi (51,700 MPa) and an elongation of approximately 5% to approximately 13%.

In various embodiments, the magnesium is overmolded in an injection molded process with a Zytel® or similar material (from DuPont of Wilmington, DE) shell. The Zytel® is fire resistant and provides a protective coating for the HANS device, as well as a soft-touch surface for comfort.

The strap of the tether is preferably made of a relatively inextensible, fibrous strap material.

In use, for example, the HANS device 100 shown in FIGS. 1-4, is attached onto the shoulder of a driver, as partially shown in FIG. 9, such that the neck of the driver is adjacent the notch 117 of the central portion 115 of the low collar 110, the top portion 122A of the left leg 122 engages the left shoulder of the driver, the bottom portion 122B of the left leg 122 engages the left chest area of the driver, the top portion 124A of the right leg 124 engages the right shoulder of the driver, and the bottom portion 124B of the right leg 124 engages the right chest area of the driver, respectively. The strap 131 of the tether 130 is coupled to the low collar 110 through the pair of openings 112 of the low collar 110. The catches 132 are attached to posts 102 of the helmet 108 of the driver so as to engage the low collar 110 with the helmet 108 of the driver. The tether 130 is connected between the low collar 110 and the helmet 108. According to the invention, the first connection of the tether 130 to the helmet 108 at the helmet end (i.e., at the posts 102) is below the horizontal level of the center of gravity (HCG) of the combined driver's head and helmet and behind the vertical line of the center of gravity (VCG) of the combined driver's head and helmet, while the second connection of the tether 130 to the low collar 110 at the low collar end (i.e., at the openings 112) is lower than the first connection, such that the tether 130 is essentially at an angle, α, relative to the HCG, as shown in FIG. 9. In other words, in operation, the combined driver's head and helmet center of gravity is significantly above the low collar 110 of the HANS device 100.

The HANS device 100 is securely held in place by the shoulder belts of the shoulder harness (not shown) when the driver is securely belted into the vehicle. The HANS device 100 is only secured to the driver of the vehicle by the shoulder belts. This allows the driver to exit the vehicle without having to remove the HANS device 100. The shoulder belts of the shoulder harness extend along the load bearing surfaces (or thin film/layer) 142 and 144 on the left and right legs 122 and 124 such that the left and right legs 122 and 124 are between the shoulder belts and the driver and the load bearing surfaces 142 and 144 are above the driver's shoulders. The load bearing surfaces 142 and 144 at the top (shoulder) portions 122A and 124A of the left and right legs 122 and 124 are positioned such that the shoulder belts of the shoulder harness adjacent the top (shoulder) portions 122A and 124A are substantially parallel with the horizontal level of the top of the shoulders of the driver. The shoulder belts of the shoulder harness hold the yoke structure securely in contact with the front of the chest and the shoulders of the driver during normal vehicle operation. During a crash, the connection of the tethers 130 to the helmet 108 tends to pull the entire HANS device 100 forward and the shoulder portions 122A and 124A of the left and right legs 122 and 124 and the low collar 110 of the HANS device 100 upward. During a crash, the tether 130 carries tension forces from the helmet 108 to the low collar 110 of the HANS device 100.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its scope. Accordingly, the scope of the present invention is defined by the appended claims rather than the foregoing description and the exemplary embodiments described therein.

## Claims

1. A head and neck support (HANS) device (100; 200), comprising:
a low collar (110; 210) having a left side (111), a right side (113), a central portion (115; 215) defined between the left side and the right side, and a pair of openings (112; 212) formed in the central portion (115; 215) and proximate to the left side and the right side, respectively;
a left leg (122; 222) having a top portion (122A) extending laterally and outwardly from the left side of the low collar (110; 210), and a bottom portion (122B) extending curvedly and downwardly from the top portion (122A); and
a right leg (124; 224) having a top portion (124A) extending laterally and outwardly extending from the right side of the low collar (110; 210), and a bottom portion (124B) extending curvedly and downwardly from the top portion (124A),
wherein the low collar (110; 210), the left leg and the right leg (124; 224), integrally form a yoke structure therewith, such that the central portion of the low collar (110; 210) is operable to be positioned behind the neck of a driver, the top portion (122A) of the left leg (122; 222) is operable to engage the left shoulder of the driver, the bottom portion (122B) of the left leg (122; 222) is operable to engage the left chest area of the driver, the top portion (124A) of the right leg (124; 224) is operable to engage the right shoulder of the driver, and the bottom portion (124B) of the right leg (124; 224) is operable to engage the right chest area of the driver, respectively;
**characterized in that** the low collar (110; 210) is characterized with a distance between a horizontal line (119) crossing the middle of the pair of openings (112) of the low collar (110; 210) and a top end of the top portion (122A; 124A) of each of the left leg (122; 222) and the right leg (124; 224, 324), wherein the distance (h) is less than 70 mm; and
a tether (130) having a strap (131) coupled to the low collar (110; 210) through the pair of openings of the low collar (110; 210), and a pair of catches (132) connected to two ends of the strap (131), wherein the catches of the tether (130) are operable to attach to a helmet of the driver so as to engage the low collar (110; 210) with the helmet of the driver.

2. A head and neck support (HANS) device (300), comprising:
a low collar (310) having a left side, a right side, a central portion (315) defined between the left side and the right side, a rear surface (310B) and at least one sleeve member (312) attached to the rear surface (310B) to define an opening (312A) therewith;
a left leg (322) having a top portion extending laterally and outwardly from the left side of the low collar (310), and a bottom portion extending curvedly and downwardly from the top portion; and
a right leg (324) having a top portion extending laterally and outwardly extending from the right side of the low collar (310), and a bottom portion extending curvedly and downwardly from the top portion,
wherein the low collar (310), the left leg and the right leg (324) integrally form a yoke structure therewith, such that the central portion of the low collar (310) is operable to be positioned behind the neck of a driver, the top portion of the left leg (322) is operable to engage the left shoulder of the driver, the bottom portion of the left leg (322) is operable to engage the left chest area of the driver, the top portion (124A) of the right leg (124; 224, 324) is operable to engage the right shoulder of the driver, and the bottom portion of the right leg (324) is operable to engage the right chest area of the driver, respectively;
**characterized in that** the low collar (310) is characterized with a distance between a horizontal line crossing the middle of the opening (312A) of the at least one sleeve member (312) and a top end of the top portion of each of the left leg (322) and the right leg (324), wherein the distance (h) is less than 70 mm; and
a tether (130) having a strap (131) coupled to the low collar (310) through the opening (312A) of the at least one sleeve member (312), and a pair of catches (132) connected to two ends of the strap (131), wherein the catches of the tether (130) are operable to attach to a helmet of the driver so as to engage the low collar (310) with the helmet of the driver.

3. The HANS device (100; 200; 300) of claim 1 or 2, **characterized in that** the low collar (110; 210; 310) is formed such that a portion of the strap (131) of the tether is exposed at a rear surface of the low collar (110; 210; 310).

4. The HANS device (213A; 200) of claim 1, **characterized in that** the low collar (210) has a recess (213A) formed on a rear surface of the low collar (210) at the central portion of the low collar (210) such that the pair of openings (212) are located in the recess, and a peg member configured complementarily to the recess (213A) and operable to be received in the recess, and wherein as assembled, the strap (131) of the tether (130) passes through the pair of openings (212) of the low collar (210) and a portion of the strap is accommodated in the recess of the low collar (210), and the peg member (213B) is pegged into the recess (213A), such that no portion of the strap of the tether (130) is visually exposed in a rear view of the low collar (210).

5. The HANS device (100; 200; 300) of claims 1 or 2, **characterized in that** the low collar (110; 210; 310) has a curved notch formed on a bottom edge of the central portion (115; 215; 315), wherein the notch is operable to accommodate the neck of the driver.

6. The HANS device (100; 200; 300) of claims 1 or 2, **characterized in that** the low collar (110; 210; 310) has a collar height that varies gradually from a central portion (115; 215; 315) to the left and right sides, wherein the collar height at the central portion (115; 215; 315) is higher than that at the left and right sides.

7. The HANS device (100; 200; 300) of claims 1 or 2 further comprising a left cushion detachably attached to a bottom surface of the left leg (122; 222; 322), and a right cushion detachably attached to a bottom surface of the right leg (124; 224; 324), **characterized in that** each cushion is operable to provide a buffer action for the corresponding shoulder and chest area of the driver.

8. The HANS device (100; 200; 300) of claims 1 or 2, further comprising a left load-bearing thin layer formed on a top surface of the left leg (122; 222; 322), and a right load-bearing thin layer formed on a top surface of the right leg (124; 224; 324), **characterized in that** each load-bearing thin layer is formed of a friction material and is operable to resist the shoulder belts from slipping on the load bearing surfaces of the HANS device.

9. The HANS device (100; 200; 300) of claims 1 or 2, **characterized in that** a top surface of each of the left leg (122; 222; 322) and the right leg (124; 224; 324) is provided with a friction means, wherein the friction means is operable to provide for better transference of force from the load bearing surfaces to the shoulder harness.

10. The HANS device (100; 200; 300) of claims 1 or 2, **characterized in that** each of the left leg (122; 222; 322) and the right leg (124; 224; 324) has an upward lip with a variable height extending upwardly from an exterior edge of the top portion of the corresponding leg, wherein the upward lip is operable to reduce the chance of a shoulder belt of a safety harness sliding off the HANS device (100; 200; 300).

## Patentansprüche

1. Eine Kopf- und Nackenstützvorrichtung (HANS) (100; 200), umfassend:
einen niedrigen Kragen (110; 210) mit einer linken Seite (111), einer rechten Seite (113), einem zentralen Abschnitt (115; 215), der zwischen der linken Seite und der rechten Seite definiert ist, und einem Paar von Öffnungen (112; 212), die in dem zentralen Abschnitt (115; 215) ausgebildet sind und jeweils nahe der linken Seite und der rechten Seite liegen;
einen linken Schenkel (122; 222) mit einem oberen Abschnitt (122A), der sich seitlich und nach außen von der linken Seite des niedrigen Kragens (110; 210) erstreckt, und einem unteren Abschnitt (122B), der sich gekrümmt und nach unten von dem oberen Abschnitt (122A) erstreckt; und
einen rechten Schenkel (124; 224) mit einem oberen Abschnitt (124A), der sich seitlich und nach außen erstreckt und sich von der rechten Seite des niedrigen Kragens (110; 210) erstreckt, und einem unteren Abschnitt (124B), der sich gekrümmt und nach unten von dem oberen Abschnitt (124A) erstreckt,
wobei der niedrige Kragen (110; 210), der linke Schenkel und der rechte Schenkel (124; 224) integral eine Jochstruktur damit bilden, so dass der mittlere Abschnitt des niedrigen Kragens (110; 210) betreibbar ist, um hinter dem Hals eines Fahrers positioniert zu werden, der obere Abschnitt (122A) des linken Schenkels (122; 222) operabel ist, um in die linke Schulter des Fahrers einzugreifen, der untere Abschnitt (122B) des linken Schenkels (122; 222) ist betreibbar, um in den linken Brustbereich des Fahrers einzugreifen, der obere Abschnitt (124A) des rechten Schenkels (124; 224) ist betreibbar, um in die rechte Schulter des Fahrers einzugreifen, und der untere Abschnitt (124B) des rechten Schenkels (124; 224) ist betreibbar, um in den rechten Brustbereich des Fahrers einzugreifen, jeweils;
**dadurch gekennzeichnet, dass** der niedrige Kragen (110; 210) mit einem Abstand zwischen einer horizontalen Linie (119), die die Mitte des Paares von Öffnungen (112) des niedrigen Kragens (110; 210) kreuzt, und einem oberen Ende des oberen Abschnitts (122A; 124A) jedes des linken Schenkels (122; 222) und des rechten Schenkels (124; 224, 324) ist, wobei der Abstand (h) kleiner als 70 mm ist; und
ein Haltegurt (130) mit einem Gurt (131), der mit dem niedrigen Kragen (110; 210) durch das Paar von Öffnungen des niedrigen Kragens (110; 210) gekoppelt ist, und einem Paar von Verschlüssen (132), die mit zwei Enden des Gurtes (131) verbunden sind, wobei die Verschlüsse des Haltegurtes (130) betreibbar sind, um an einem Helm des Fahrers befestigt zu werden, um so den niedrigen Kragen (110; 210) mit dem Helm des Fahrers in Eingriff zu bringen.

2. Eine Kopf- und Nackenstützvorrichtung (HANS) (300), umfassend:
einen niedrigen Kragen (310) mit einer linken Seite, einer rechten Seite, einem zentralen Abschnitt (315), der zwischen der linken Seite und der rechten Seite definiert ist, einer hinteren Fläche (310B) und mindestens einem Hülsenelement (312), das an der hinteren Fläche (310B) befestigt ist, um eine Öffnung (312A) damit zu definieren;
einen linken Schenkel (322) mit einem oberen Abschnitt, der sich seitlich und nach außen von der linken Seite des niedrigen Kragens (310) erstreckt, und einem unteren Abschnitt, der sich gekrümmt und nach unten von dem oberen Abschnitt erstreckt; und
einen rechten Schenkel (324) mit einem oberen Abschnitt, der sich seitlich und nach außen von der rechten Seite des niedrigen Kragens (310) erstreckt, und einem unteren Abschnitt, der sich gekrümmt und nach unten von dem oberen Abschnitt erstreckt,
wobei der niedrige Kragen (310), der linke Schenkel und der rechte Schenkel (324) integral eine Jochstruktur damit bilden, so dass der mittlere Abschnitt des niedrigen Kragens (310) betreibbar ist, um hinter dem Hals eines Fahrers positioniert zu werden, der obere Abschnitt des linken Schenkels (322) betreibbar ist, um in die linke Schulter des Fahrers einzugreifen, der untere Abschnitt des linken Schenkels (322) betreibbar ist, um in den linken Brustbereich des Fahrers einzugreifen, der obere Abschnitt (324A) des rechten Schenkels (324) ist betreibbar, um die rechte Schulter des Fahrers einzugreifen, und der untere Abschnitt des rechten Schenkels (324) ist betreibbar, um den rechten Brustbereich des Fahrers einzugreifen;
**dadurch gekennzeichnet, dass** der niedrige Kragen (310) mit einem Abstand zwischen einer horizontalen Linie, die die Mitte der Öffnung (312A) des mindestens einen Hülsenelements (312) kreuzt, und einem oberen Ende des oberen Abschnitts jedes des linken Schenkels (322) und des rechten Schenkels (324) ist, wobei der Abstand (h) weniger als 70 mm beträgt; und
ein Haltegurt (130) mit einem Gurt (131), der mit dem niedrigen Kragen (310) durch die Öffnung (312A) des mindestens einen Hülsenelements (312) gekoppelt ist, und einem Paar Verschlüsse (132), die mit zwei Enden des Gurtes (131) verbunden sind, wobei die Verschlüsse des Haltegurtes (130) betreibbar sind, um an einem Helm des Fahrers befestigt zu werden, so dass der niedrige Kragen (310) mit dem Helm des Fahrers eingegriffen wird.

3. HANS-Vorrichtung (100; 200; 300) nach Anspruch 1 oder 2, **dadurch gekennzeichnet, dass** der niedrige Kragen (110; 210; 310) so ausgebildet ist, dass ein Abschnitt des Gurts (131) des Haltergurts an einer Rückseite des niedrigen Kragens (110; 210; 310) freigelegt ist.

4. HANS-Vorrichtung (213A; 200) nach Anspruch 1, **dadurch gekennzeichnet, dass** der niedrige Kragen (210) eine Aussparung (213A) aufweist, die auf einer Rückseite des niedrigen Kragens (210) am mittleren Abschnitt des niedrigen Kragens (210) so ausgebildet ist, dass sich das Paar von Öffnungen (212) in der Aussparung befindet, und ein Zapfenelement, das komplementär zu der Aussparung (213A) ausgebildet und betreibbar in der Aussparung aufzunehmen ist, und wobei im montierten Zustand, der Gurt (131) des Haltegurts (130) durch das Paar von Öffnungen (212) des niedrigen Kragens (210) verläuft und ein Abschnitt des Gurts in der Aussparung des niedrigen Kragens (210) aufgenommen ist, und das Zapfenelement (213B) in die Aussparung (213A) eingesteckt ist, so dass kein Abschnitt des Gurts des Haltegurts (130) in einer Rückansicht des niedrigen Kragens (210) visuell freigelegt ist.

5. HANS-Vorrichtung (100; 200; 300) nach Anspruch 1 oder 2, **dadurch gekennzeichnet, dass** der niedrige Kragen (110; 210; 310) eine gekrümmte Kerbe aufweist, die an einer Unterkante des zentralen Abschnitts (115; 215; 315) ausgebildet ist, wobei die Kerbe betreibbar ist, um den Hals des Fahrers aufzunehmen.

6. HANS-Vorrichtung (100; 200; 300) nach Anspruch 1 oder 2, **dadurch gekennzeichnet, dass** der niedrige Kragen (110; 210; 310) eine Kragenhöhe aufweist, die allmählich von einem zentralen Abschnitt (115; 215; 315) nach links und rechts variiert, wobei die Kragenhöhe am zentralen Abschnitt (115; 215; 315) höher ist als die am linken und rechten Rand.

7. HANS-Vorrichtung (100; 200; 300) nach Anspruch 1 oder 2, ferner umfassend ein linkes Kissen, das lösbar an einer Unterseite des linken Schenkels (122; 222; 322) befestigt ist, und ein rechtes Kissen, das lösbar an einer Unterseite des rechten Schenkels (124; 224; 324) befestigt ist, **dadurch gekennzeichnet, dass** jedes Kissen betreibbar ist, um eine Pufferwirkung für den entsprechenden Schulter- und Brustbereich des Fahrers bereitzustellen.

8. HANS-Vorrichtung (100; 200; 300) nach Anspruch 1 oder 2, ferner umfassend eine linke lasttragende dünne Schicht, die auf einer Oberseite des linken Schenkels (122; 222; 322) ausgebildet ist, und eine rechte lasttragende dünne Schicht, die auf einer Oberseite des rechten Schenkels (124; 224; 324) ausgebildet ist, **dadurch gekennzeichnet, dass** jede lasttragende dünne Schicht aus einem Reibmaterial gebildet und so betreibbar ist, dass sie den Schultergurten ein Verrutschen auf den lasttragenden Oberflächen der HANS-Vorrichtung verhindert.

9. HANS-Vorrichtung (100; 200; 300) nach Anspruch 1 oder 2, **dadurch gekennzeichnet, dass** eine Oberseite jedes des linken Schenkels (122; 222; 322) und des rechten Schenkels (124; 224; 324) mit einem Reibungsmittel versehen ist, wobei das Reibungsmittel betreibbar ist, um eine bessere Kraftübertragung von den lasttragenden Oberflächen auf das Schultergeschirr zu gewährleisten.

10. HANS-Vorrichtung (100; 200; 300) nach Anspruch 1 oder 2, **dadurch gekennzeichnet, dass** jedes des linken Schenkels (122; 222; 322) und des rechten Schenkels (124; 224; 324) eine Aufwärtslippe mit einer variablen Höhe aufweist, die sich von einer Außenkante des oberen Abschnitts des entsprechenden Schenkels nach oben erstreckt, wobei die Aufwärtslippe betreibbar ist, um die Wahrscheinlichkeit zu verringern, dass ein Schultergurt eines Sicherheitsgeschirrs von der HANS-Vorrichtung (100; 200; 300) gleitet.

## Revendications

1. Dispositif de support de tête et de cou (STEC) (100 ; 200), comprenant :
un col bas (110 ; 210) ayant un côté gauche (111), un côté droit (113), une partie centrale (115 ; 215) définie entre le côté gauche et le côté droit, et une paire d'ouvertures (112 ; 212) formées dans la partie centrale (115 ; 215) et à proximité du côté gauche et du côté droit, respectivement ;
une patte gauche (122, 222) ayant une partie supérieure (122A) s'étendant de manière latérale et vers l'extérieur à partir du côté gauche du col bas (110 ; 210), et
une partie inférieure (122B) s'étendant de manière incurvée et vers le bas à partir de la partie supérieure (122A) ; et
une patte droite (124 ; 224) ayant une partie supérieure (124A) s'étendant de manière latérale et vers l'extérieur à partir du côté droit du col bas (110 ; 210), et
une partie inférieure (124B) s'étendant de manière incurvée et vers le bas à partir de la partie supérieure (124A),
dans lequel le col bas (110 ; 210), la patte gauche et la patte droite (124 ; 224) forment ensemble une structure de rotule d'un seul tenant, de telle sorte que la partie centrale du col bas (110 ; 210) peut fonctionner de façon à être positionnée derrière le cou d'un conducteur, la partie supérieure (122A) de la patte gauche (122 ; 222) peut fonctionner de façon à entrer en prise avec l'épaule gauche du conducteur, la partie inférieure (122B) de la patte gauche (122 ; 222) peut fonctionner de façon à entrer en prise avec la région de torse gauche du conducteur, la partie supérieure (124A) de la patte droite (124 ; 224) peut fonctionner de façon à entrer en prise avec l'épaule droite du conducteur, et la partie inférieure (124B) de la patte droite (124 ; 224) peut fonctionner de façon à entrer en prise avec la région de torse droite du conducteur, respectivement ;
**caractérisé en ce que** le col bas (110 ; 210) est **caractérisé par** une distance entre une ligne horizontale (119) traversant le milieu de la paire d'ouvertures (112) du col bas (110 ; 210) et une extrémité supérieure de la partie supérieure (122A ; 124A) de chacune des pattes de la patte gauche (122 ; 222) et de la patte droite (124 ; 224 ; 324), dans lequel la distance (h) est inférieure à 70 mm ; et
une attache (130) ayant une sangle (131) couplée au col bas (110 ; 210) par l'intermédiaire de la paire d'ouvertures du col bas (110 ; 210), et une paire de prises (132) reliées à deux extrémités de la sangle (131), dans lequel les prises de l'attache (130) peuvent fonctionner pour se fixer à un casque du conducteur de façon à amener le col bas (110 ; 210) en prise avec le casque du conducteur.

2. Dispositif de support de tête et de cou (STEC) (300), comprenant :
un col bas (310) ayant un côté gauche, un côté droit, une partie centrale (315) définie entre le côté gauche et le côté droit, une surface arrière (310B) et au moins un élément de manchon (312) fixé à la surface arrière (310B) pour définir une ouverture (312A) avec celle-ci ;
une patte gauche (322) ayant une partie supérieure s'étendant de manière latérale et vers l'extérieur à partir du côté gauche du col bas (310) et une partie inférieure s'étendant de manière incurvée et vers le bas à partir de la partie supérieure ; et
une patte droite (324) ayant une partie supérieure s'étendant de manière latérale et vers l'extérieur à partir du côté droit du col bas (310), et une partie inférieure s'étendant de manière incurvée et vers le bas à partir de la partie supérieure,
dans lequel le col bas (310), la patte gauche et la patte droite (324) forment ensemble une structure de rotule d'un seul tenant, de telle sorte que la partie centrale du col bas (310) peut fonctionner de façon à être positionnée derrière le cou d'un conducteur, la partie supérieure de la patte gauche (322) peut fonctionner de façon à entrer en prise avec l'épaule gauche du conducteur, la partie inférieure de la patte gauche (322) peut fonctionner de façon à entrer en prise avec la région de torse gauche du conducteur, la partie supérieure (124A) de la patte droite (124 ; 224 ; 324) peut fonctionner de façon à entrer en prise avec l'épaule droite du conducteur, et la partie inférieure de la patte droite (324) peut fonctionner de façon à entrer en prise avec la région de torse droite du conducteur, respectivement ;
**caractérisé en ce que** le col bas (310) est **caractérisé par** une distance entre une ligne horizontale traversant le milieu des ouvertures (312A) dudit un élément de manchon (312) et une extrémité supérieure de la partie supérieure de chacune des pattes de la patte gauche (322) et de la patte droite (324), dans lequel la distance (h) est inférieure à 70 mm ; et
une attache (130) ayant une sangle (131) couplée au col bas (310) par l'intermédiaire des ouvertures (312A) dudit au moins un élément de manchon (312), et une paire de prises (132) reliées à deux extrémités de la sangle (131), dans lequel les prises de l'attache (130) peuvent fonctionner pour se fixer à un casque du conducteur de façon à amener le col bas (310) en prise avec le casque du conducteur.

3. Dispositif STEC (100 ; 200 ; 300) selon la revendication 1, ou 2, **caractérisé en ce que** le col bas (110 ; 210 ; 310) est formé de telle sorte qu'une partie de la sangle (131) de l'attache est exposée à une surface arrière du col bas (110 ; 210 ; 310).

4. Dispositif STEC (213A ; 200) selon la revendication 1, **caractérisé en ce que** le col bas (210) a un renfoncement (213A) formé sur une surface arrière du col bas (210) au niveau de la partie centrale du col bas (210) de telle sorte que la paire d'ouvertures (212) est située dans le renfoncement, et un élément de cheville configuré de manière complémentaire au renfoncement (213A) et pouvant fonctionner pour être reçu dans le renfoncement, et dans lequel, une fois assemblé, la sangle (131) de l'attache (130) passe à travers la paire d'ouvertures (212) du col bas (210) et une partie de la sangle est reçue dans le renfoncement du col bas (210), et l'élément de cheville (213B) est enfoncé à l'intérieur du renfoncement (213A) de telle sorte qu'aucune partie de la sangle de l'attache (130) n'est exposée visuellement dans une vue arrière du col bas (210).

5. Dispositif STEC (100 ; 200 ; 300) selon la revendication 1 ou 2, **caractérisé en ce que** le col bas (110 ; 210 ; 310) a une encoche incurvée formée sur un bord inférieur de la partie centrale (115 ; 215 ; 315), dans lequel l'encoche peut fonctionner pour recevoir le cou du conducteur.

6. Dispositif STEC (100 ; 200 ; 300) selon la revendication 1 ou 2, **caractérisé en ce que** le col bas (110 ; 210 ; 310) a une hauteur de col qui varie progressivement d'une partie centrale (115 ; 215 ; 315) vers les côtés gauche et droit, dans lequel la hauteur du col au niveau de la partie centrale (115 ; 215 ; 315) est supérieure à celle au niveau des côtés gauche et droit.

7. Dispositif STEC (100 ; 200 ; 300) selon la revendication 1 ou 2, comprenant en outre un coussinet gauche fixé de manière amovible à une surface inférieure de la patte gauche (122 ; 222 ; 322) et un coussinet droit fixé de manière amovible à une surface inférieure de la patte droite (124 ; 224 ; 324), **caractérisé en ce que** chaque coussinet peut fonctionner de façon à fournir une action d'amortissement à l'épaule et à la région de torse correspondantes du conducteur.

8. Dispositif STEC (100 ; 200 ; 300) selon la revendication 1 ou 2, comprenant en outre une couche fine porteuse de charge gauche formée sur une surface supérieure de la patte gauche (122 ; 222 ; 322) et une couche fine porteuse de charge droite formée sur une surface supérieure de la patte droite (124 ; 224 ; 324), **caractérisé en ce que** chaque couche fine porteuse de charge est formée à partir d'un matériau de friction et peut fonctionner pour empêcher les ceintures d'épaule de glisser sur les surfaces porteuses de charge du dispositif STEC.

9. Dispositif STEC (100 ; 200 ; 300) selon la revendication 1 ou 2, **caractérisé en ce qu'**une surface supérieure de chacune des pattes de la patte gauche (122 ; 222 ; 322) et de la patte droite (124 ; 224 ; 324) est pourvue d'un moyen de friction, dans lequel le moyen de friction peut fonctionner pour fournir une meilleure transmission de force des surfaces porteuses de charge au harnais d'épaule.

10. Dispositif STEC (100 ; 200 ; 300) selon la revendication 1 ou 2, **caractérisé en ce que** chacune des pattes de la patte gauche (122 ; 222 ; 322) et de la patte droite (124 ; 224 ; 324) a une lèvre verticale avec une hauteur variable s'étendant vers le haut à partir d'un bord extérieur de la partie supérieure de la patte correspondante, dans lequel la lèvre verticale peut fonctionner pour réduire le risque qu'une ceinture d'épaule d'un harnais de sécurité glisse hors du dispositif STEC (100 ; 200 ; 300).
